# EUROPEAN PATENT APPLICATION

(11) **EP 4 056 188 A1**
(43) Date of publication of application: **14.09.2022**
(21) Application number: 20883890.4
(22) Date of filing: 06.11.2020
(51) Int. Cl.: A61K 33/00, A61M 15/00, A61M 16/00, A61P 9/06

(54) **AGENT FOR PREVENTING OR TREATING ARRHYTHMIA AND DEVICE FOR PREVENTING OR TREATING ARRHYTHMIA**

(30) Priority: 08.11.2019 JP 2019203385
(71) Applicant: School Juridical Person The Kitasato Institute, Tokyo 108-8641 (JP)
(72) Inventor: KOKUBO Kenichi, Sagamihara-shi, Kanagawa 252-0373 (JP); KOBAYASHI Hirosuke, Sagamihara-shi, Kanagawa 252-0373 (JP); AKO Junya, Sagamihara-shi, Kanagawa 252-0373 (JP)
(74) Representative: Regimbeau
(86) International application number: PCT/JP2020/041586
(87) International publication number: WO 2021/090926

(57) **Abstract**

An agent for preventing or treating an arrhythmia, which contains hydrogen.

## Description

### Field of the Invention

The present invention relates to an agent for preventing or treating an arrhythmia and a device for preventing or treating an arrhythmia.

Priority is claimed on Japanese Patent Application No. 2019-203385, filed November 8, 2019, the content of which is incorporated herein by reference.

### Description of Related Art

A sinus rhythm is a condition in which electrical excitement generated in the sinus node present in the right atrium of the heart is correctly transmitted to the atrium, atrioventricular node, and ventricle and repeated at a certain rhythm. An arrhythmia is a condition in which the heart deviates from a regular sinus rhythm.

An arrhythmia that occurs while an acute myocardial infarction occurs, after an acute myocardial infarction is treated, and after rehabilitation is performed is often fatal. Therefore, it is conceivable that, if an arrhythmia can be prevented, unexpected death can be reduced.

In the related art, a sodium channel blocker, a β-adrenaline blocker, a calcium antagonist and the like have been used as arrhythmia-treating agents. For example, as exemplified in Patent Document 1, most arrhythmia-treating agents are low-molecular-weight compounds.

### Citation List

### [Patent Documents]

[Patent Document 1] Japanese Patent No. 5039236

### Summary of the Invention

### Technical Problem

However, administration of a conventional arrhythmia-treating agent may cause side effects, for example, hypoglycemia, liver disorders, and skin disorders, and further improvement is required. Therefore, an object of the present invention is to provide a technique for preventing or treating an arrhythmia, which has a low risk of side effects and can be performed simply.

### Solution to Problem

The present invention includes the following aspects.
[1] An agent for preventing or treating an arrhythmia, comprising hydrogen.
[2] The agent for preventing or treating an arrhythmia according to [1], further comprising nitric oxide.
[3] A device for preventing or treating an arrhythmia which includes a hydrogen gas supply source, a pressure regulator and a flowmeter connected to the hydrogen gas supply source, and a supply path through which the hydrogen gas is supplied from the supply source, and administers the agent for preventing or treating an arrhythmia according to [1] to a patient by inhalation in a direct manner or via an artificial respirator.
[4] A device for preventing or treating an arrhythmia which includes a hydrogen gas supply source and a nitric oxide gas supply source, a pressure regulator and a flowmeter connected to the hydrogen gas supply source and the nitric oxide gas supply source, and supply paths through which respective gases are supplied from the supply sources, and in which all gas supply paths are disposed so that they merge before administration to form a mixed gas, and which administers the agent for preventing or treating an arrhythmia according to [2] to a patient by inhalation in a direct manner or via an artificial respirator.

### Advantageous Effects of Invention

According to the present invention, it is possible to provide a technique for preventing or treating an arrhythmia, which has a low risk of side effects and can be performed simply.

### Brief Description of the Drawings

FIG. 1 is a schematic diagram showing an example of a device for preventing or treating an arrhythmia.
FIG. 2 is a schematic diagram showing an example of a device for preventing or treating an arrhythmia.
FIG. 3 is a graph showing the results of Experiment Example 1.

### Detailed Description of the Invention

Hereinafter, embodiments of the present invention will be described in detail with reference to the drawings in some cases. Here, in the drawings, the same or corresponding parts will be denoted with the same or corresponding reference numerals, and redundant descriptions thereof will be omitted. Here, the dimensional ratios in the drawings may be exaggerated for explanation, and do not necessarily match actual dimensional ratios.

### [Agent for preventing or treating arrhythmia]

In one embodiment, the present invention provides an agent for preventing or treating an arrhythmia, which contains hydrogen. The agent for preventing or treating an arrhythmia of the present embodiment contains hydrogen as an active component. Hydrogen is also recognized as a food additive, and the safety of hydrogen dissolved in water has been confirmed. In addition, gaseous hydrogen has also been used as a gas for diving, and it has been confirmed that there are no side effects even if high-concentration hydrogen is inhaled. At this time, no articles regarding the side effects of dissolved hydrogen and hydrogen gas have been reported and hydrogen has a very low risk of side effects.

As will be described below in examples, the inventors have found that an arrhythmia can be prevented or treated by administering hydrogen. According to the preventing agent or treating agent of the present embodiment, it is possible to achieve a technique for preventing or treating an arrhythmia, which has a low risk of side effects and is simple.

The agent for preventing or treating an arrhythmia of the present embodiment may be a gas or a liquid. If the preventing agent or treating agent of the present embodiment is a gas, for example, a patient with a myocardial infarction inhales the preventing agent or treating agent of the present embodiment in an ambulance, and thus an arrhythmia can be prevented. In addition, for example, in heart rehabilitation, exercise therapy can be performed while preventing an arrhythmia by inhaling the preventing agent or treating agent of the present embodiment.

If the preventing agent or treating agent of the present embodiment is a gas, the hydrogen concentration is preferably 1 to 10% (v/v), more preferably 1 to 6% (v/v), and particularly preferably 1.5 to 4% (v/v). Examples of components other than hydrogen in the gas include an inert gas, oxygen, and additives. Examples of inert gases include nitrogen. Examples of additives include nitric oxide, fragrances and the like which will be described below. If the preventing agent or treating agent of the present embodiment is a gas, it may be administered by inhalation through the patient's mouth, nose or the like, or may be administered by inhalation to the patient's lungs through an artificial respirator.

In addition, the preventing agent or treating agent of the present embodiment may be a liquid in which hydrogen is dissolved. Examples of liquids include water, a physiological saline solution, and a buffer solution. For example, when the maximum saturation of hydrogen in hydrogen water under 1 atm is 1.57 ppm, the hydrogen concentration in the liquid preventing agent or treating agent is preferably 1 ppm or more, more preferably 1.2 ppm or more, and particularly preferably 1.5 ppm or more.

If the preventing agent or treating agent of the present embodiment is a liquid, the liquid may further contain a stabilizer, a preservative, a pH-regulating agent, a colorant, a fragrance and the like as pharmaceutically acceptable additives. Examples of stabilizers include parahydroxybenzoic acid esters such as methylparaben and propylparaben; alcohols such as chlorobutanol; and phenols such as phenol and cresol. Examples of pH-regulating agents include phthalic acid, phosphoric acid, citric acid, succinic acid, acetic acid, fumaric acid, malic acid, carbonic acid and their potassium salts, sodium salts or ammonium salts, and sodium hydroxide.

If the preventing agent or treating agent of the present embodiment is a liquid, it may be administered orally to a patient or may be administered by intra-arterial injection, intravenous injection, subcutaneous injection or the like.

The preventing agent or treating agent of the present embodiment may further contain nitric oxide. In infarction diseases such as a myocardial infarction, a cerebral infarction, and mesenteric vaso-occlusive diseases, ischemia-reperfusion therapy in which an infarcted area is mechanically dilated or thrombi are dissolved and thus blood flow is restored to an ischemic tissue in which the blood flow has been interrupted may be performed. In addition, in transplanted organs after organ transplantation, blood flow is similarly restored to tissue in which the blood flow has been interrupted. It is known that, when this blood flow is restored, since oxygen is suddenly added to the ischemic tissue, various physiological reactions such as production of active oxygen and free radicals, infiltration of neutrophils, and platelet activation occur, which exacerbate the organ damage, and this is called an ischemia-reperfusion disorder.

It is known that nitric oxide has a function of dilating blood vessels and can reduce ischemia-reperfusion disorders according to administration. However, nitric oxide reacts with superoxide anions produced inside and outside cells to produce peroxynitrite, which is extremely highly damaging to tissue, and has a high risk of side effects. On the other hand, it is known that hydrogen reacts with peroxynitrite and active oxygen species to reduce and eliminate them.

Here, when the preventing agent or treating agent of the present embodiment further contains nitric oxide, nitrous acid or a nitric oxide donor, blood vessels of the lungs and the heart are dilated while the side effects of nitric oxide are inhibited, and for example, exercise therapy in rehabilitation can be performed more efficiently. In addition, an arrhythmia can be inhibited and unexpected deaths can be reduced. The nitric oxide concentration in the preventing agent or treating agent of the present embodiment is preferably 1 to 100 ppm.

It is conceivable that, for example, administration at the time of heart failure exacerbation in addition to a rehabilitation time may dilate blood vessels and reduce a risk of arrhythmia, which can not only improve the pathological condition but also reduce unexpected deaths.

### [Device for preventing or treating arrhythmia]

### (First embodiment)

A device of a first embodiment includes a hydrogen gas supply source, a pressure regulator and a flowmeter connected to the hydrogen gas supply source, and a supply path through which the hydrogen gas is supplied from the supply source, and is for preventing or treating an arrhythmia by administering a hydrogen-containing agent for preventing or treating an arrhythmia to a patient by inhalation in a direct manner or via an artificial respirator.

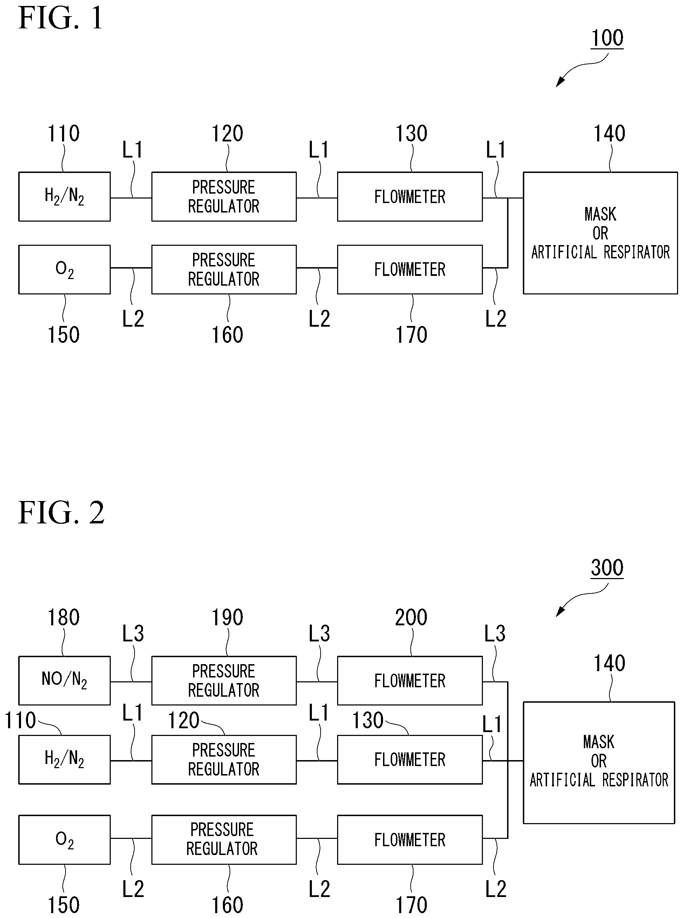

FIG. 1 is a schematic diagram showing an example of the device of the first embodiment. As shown in FIG. 1, a device 100 of the first embodiment includes a hydrogen gas supply source 110, a pressure regulator 120 and a flowmeter 130 connected to the hydrogen gas supply source 110, and a supply path L1 through which hydrogen gas is supplied from the supply source 110. A valve (not shown) for controlling an amount of the agent for preventing or treating an arrhythmia supplied is preferably provided at the supply path L1.

The hydrogen gas supply source 110 may include an inert gas such as nitrogen in addition to hydrogen gas, and may regulate the hydrogen concentration. In addition, for the patient's breathing, it is preferable that the device 100 further include an oxygen gas supply source 150, a pressure regulator 160 and a flowmeter 170 connected to the oxygen gas supply source 150, and a supply path L2 through which oxygen gas is supplied from the supply source 150. A valve (not shown) for controlling an amount of oxygen supplied is preferably provided at the supply path L2. The hydrogen gas supply source 110 and the oxygen gas supply source 150 may be, for example, cylinders.

According to the device 100, an agent for preventing or treating an arrhythmia, which contains hydrogen as an active component, is administered to the patient's lungs by inhalation via a mask or artificial respirator 140, and thus an arrhythmia can be prevented or treated.

### (Second embodiment)

A device of a second embodiment includes a hydrogen gas supply source and a nitric oxide gas supply source, a pressure regulator and a flowmeter connected to the hydrogen gas supply source and the nitric oxide gas supply source, and supply paths through which respective gases are supplied from the supply sources, and all gas supply paths are disposed so that they merge before administration to form a mixed gas, a nitric oxide-containing agent for preventing or treating an arrhythmia is administered to the patient by inhalation in a direct manner or via an artificial respirator, and thus an arrhythmia is prevented or treated.

The device of the second embodiment includes a nitric oxide gas supply source, a pressure regulator and a flowmeter connected to the nitric oxide gas supply source, and a supply path through which nitric oxide gas is supplied from the supply source, and this is the main difference from the device of the first embodiment.

FIG. 2 is a schematic diagram showing an example of the device of the second embodiment. As shown in FIG. 2, a device 300 of the second embodiment includes a hydrogen gas supply source 110 and a nitric oxide gas supply source 180, a pressure regulator 120 and a flowmeter 130 connected to the hydrogen gas supply source 110, a pressure regulator 190 and a flowmeter 200 connected to the nitric oxide gas supply source 180, a supply path L1 through which hydrogen gas is supplied from the supply source 110, and a supply path L3 through which nitric oxide gas is supplied from the supply source 180, and the supply paths L1 and L3 are disposed so that they merge before administration to form a mixed gas, a nitric oxide-containing agent for preventing or treating an arrhythmia is administered by inhalation via the mask or artificial respirator 140, and thus an arrhythmia is prevented or treated.

Valves (not shown) for controlling an amount of the agent for preventing or treating an arrhythmia supplied are preferably provided at the supply paths L1 and L3. In addition, the hydrogen gas supply source 110 may include an inert gas such as nitrogen in addition to hydrogen gas, and may be configured to regulate the hydrogen concentration. In addition, the nitric oxide gas supply source 180 may include an inert gas such as nitrogen in addition to nitric oxide gas, and may regulate the concentration of nitric oxide.

In addition, for the patient's breathing, it is preferable that the device 200 further include an oxygen gas supply source 150, a pressure regulator 160 and a flowmeter 170 connected to the oxygen gas supply source 150, and a supply path L2 through which oxygen gas is supplied from the supply source 150. In this case, the supply paths L1, L2 and L3 are disposed so that they merge before administration to form a mixed gas. A valve (not shown) for controlling an amount of oxygen supplied is preferably provided at the supply path L2.

The hydrogen gas supply source 110, the nitric oxide gas supply source 180, and the oxygen gas supply source 150 may be, for example, cylinders. According to the device 200, an agent for preventing or treating an arrhythmia, which contains hydrogen and nitric oxide as active components, is administered to the patient's lungs by inhalation via the mask or artificial respirator 140, and an arrhythmia can be prevented or treated.

The agent for preventing or treating an arrhythmia is generally administered for a relatively short time. Therefore, the device of the first embodiment and the device of the second embodiment can be combined with a small cylinder or an inhalation device or combined with a device that directly generates hydrogen or nitric oxide, and thus the device can be used in many places such as in a patient's home, an ambulance, and a rehabilitation room.

### [Other embodiments]

In one embodiment, the present invention provides a method for preventing or treating an arrhythmia including administering an effective amount of hydrogen to a patient who needs prevention or treatment.

In the treatment method of the present embodiment, hydrogen is the same as that described above. In addition to hydrogen, nitric oxide may be additionally administered. Nitric oxide is the same as that described above.

In one embodiment, the present invention provides hydrogen for preventing or treating an arrhythmia. Hydrogen is the same as that described above.

In one embodiment, the present invention provides a use of hydrogen for producing an arrhythmia-preventing agent or treating agent. The preventing agent or treating agent of the present embodiment may further contain nitric oxide in addition to hydrogen. In the use of the present embodiment, hydrogen is the same as that described above. In addition, nitric oxide is the same as that described above.

### Examples

Hereinafter, the present invention will be described with reference to examples, but the present invention is not limited to the following examples.

### [Experiment Example 1]

Pigs were anesthetized with pentobarbital, and electrocardiogram measurement was performed. After tracheal intubation, an artificial respirator was connected, and respiratory management was performed. A catheter was inserted into the descending aorta from the inguinal region, a balloon was placed in the anterior descending branch of the left coronary artery, and the balloon was inflated to interrupt the blood flow. After 40 minutes, an electrocardiogram was measured without doing anything for 10 minutes, the number of arrhythmias was counted, and after that, for 10 minutes, the number of arrhythmias was counted in the same manner for a control group, a hydrogen inhalation group, a nitric oxide inhalation group, or a combination group (hydrogen and nitric oxide inhalation group) in a divided manner.

A gas inhaled by the control group was a mixed gas (oxygen concentration of 35%) of oxygen and nitrogen and containing no hydrogen or nitric oxide. In addition, a gas inhaled by the hydrogen inhalation group was a mixed gas of air and hydrogen, and had a hydrogen concentration of 1.3%. In addition, a gas inhaled by the nitric oxide inhalation group was a mixed gas of air and nitric oxide and had a nitric oxide concentration of 80 ppm. In addition, a gas inhaled by the combination group was a mixed gas of air, hydrogen and nitric oxide and had a hydrogen concentration of 1.3% and a nitric oxide concentration of 80 ppm.

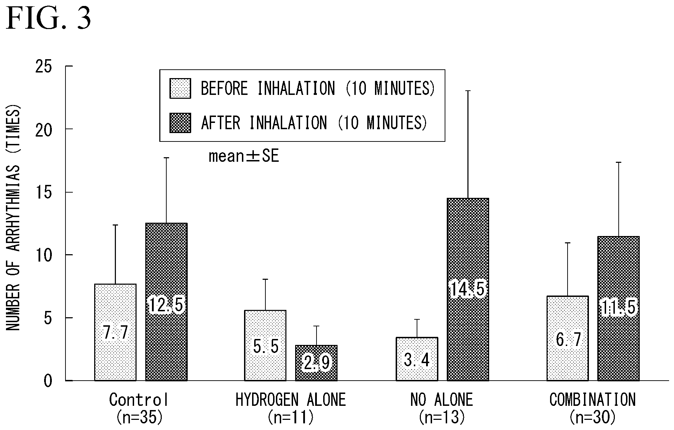

As shown in FIG. 3, in the control group, the number of arrhythmias increased, but in the hydrogen inhalation group, the number of arrhythmias decreased. In addition, it was shown from the combination group that an increase in the number of arrhythmias in the nitric oxide inhalation group could be reduced by inhaling hydrogen. Based on the above results, it can be clearly understood that an arrhythmia can be prevented or treated by administering hydrogen.

### Industrial Applicability

According to the present invention, it is possible to provide a technique for preventing or treating an arrhythmia, which has a low risk of side effects and can be performed simply.

### Reference Signs List

100, 300 Device
110 Hydrogen gas supply source
120, 160, 190 Pressure regulator
130, 170, 200 Flowmeter
L1, L2, L3 Supply path
150 Oxygen gas supply source
140 Mask or artificial respirator
180 Nitric oxide gas supply source

## Claims

1. An agent for preventing or treating an arrhythmia, comprising hydrogen.

2. The agent for preventing or treating an arrhythmia according to claim 1, further comprising nitric oxide.

3. A device for preventing or treating an arrhythmia, comprising:
a hydrogen gas supply source,
a pressure regulator and a flowmeter connected to the hydrogen gas supply source, and
a supply path through which the hydrogen gas is supplied from the supply source,
wherein the device administers the agent for preventing or treating an arrhythmia according to claim 1 to a patient by inhalation in a direct manner or via an artificial respirator.

4. A device for preventing or treating an arrhythmia, comprising:
a hydrogen gas supply source and a nitric oxide gas supply source,
a pressure regulator and a flowmeter connected to the hydrogen gas supply source and the nitric oxide gas supply source, and
supply paths through which respective gases are supplied from the supply sources,
wherein all gas supply paths are disposed so that they merge before administration to form a mixed gas, and
wherein the device administers the agent for preventing or treating an arrhythmia according to claim 2 to a patient by inhalation in a direct manner or via an artificial respirator.
